# EUROPEAN PATENT APPLICATION

(11) **EP 4 344 696 A1**
(43) Date of publication of application: **03.04.2024**
(21) Application number: 22833670.7
(22) Date of filing: 30.06.2022
(51) Int. Cl.: A61K 8/49, A61Q 7/00, A61K 31/343, A61P 17/14

(54) **FUNCTIONAL COMPOSITION FOR ALOPECIA COMPRISING CENTIPEDA MINIMA-DERIVED EXTRACT**

(30) Priority: 30.06.2021 KR 20210085581
(71) Applicant: D-Nature Co., Ltd., Seongnam-si, Gyeonggi-do 13174 (KR)
(72) Inventor: KIM, Byoungha, Hanam-si, Gyeonggi-do 13013 (KR); YUN, Sangkyu, Hwaseong-si, Gyeonggi-do 18472 (KR)
(74) Representative: BCKIP Part mbB
(86) International application number: PCT/KR2022/009433
(87) International publication number: WO 2023/277616

(57) **Abstract**

Disclosed is a composition for preventing autoimmune disease or for preventing and treating hair loss. A functional composition for hair loss according to an embodiment of the present invention is a composition for preventing and treating autoimmune disease or hair loss, the functional composition comprising a functional ingredient for hair loss extracted from a natural substance, wherein the functional ingredient for hair loss comprises, as an active ingredient, any one or more selected from the group consisting of Brevilin A, Arnicolide D, Arnicolide C, and Microhelenin C.

## Description

### Technical Field

The present invention relates to a composition for preventing and treating hair loss, and more specifically, to a composition for preventing and treating autoimmune disease or hair loss containing, as active ingredients, Brevilin A, Arnicolide D, Arnicolide C, and Microhelenin C, which are extracts from *Centipeda minima.*

### Background Art

Hair plays various roles in the human body, such as protecting the head, influencing external appearance, and maintaining the temperature of the head. Although hair is not an important organ in maintaining life, it plays an important role in forming an individual's personality and image. In addition to these aesthetic functions, hair is an important part of the body, which serves as a measure of health due to its functions such as blocking ultraviolet rays and protecting the scalp and determines appearance.

There are about 100,000 to 150,000 hairs on the human head, and each hair has a different cycle. Hair grows and falls out repeatedly through the hair cycle that includes: anagen (growing phase) which constitutes 90% of normal hair; catagen (transitional phase) in which hair growth stops and hair roots atrophy; and telogen (resting phase) in which the hair bulb dries and hair becomes club hair. This cycle is repeated over 3 to 6 years, and as a result, an average of 50 to 100 hairs are shed per day.

Hair is composed of protein, melanin pigment, lipid, trace elements, moisture, etc., and the protein, which accounts for 80 to 90% of the total, is mainly composed of keratin. Keratin is composed of 18 amino acids. The 18 amino acids found in hair form the structure of hair, mainly through three types of bonds: hydrogen bonds, which are bonds of hydrogen, ionic bonds, which are bonds of salts, and cysteine bonds, which are bonds via sulfur. Hair growth occurs through a complex mechanism caused by various genes, growth factors and their receptors, and the actions of systemic hormones. Human hair dermal papilla cells are cells that are involved in the development and growth of hair and the development of hair follicles and form the base of hair follicles together with keratinocyte cells. In addition, keratinocyte cells receive nutrition from capillaries and dermal papilla and continuously divide and proliferate to create hair shafts and hair follicles, forming hair. The formed hair is pushed up closer to the hair follicle. While the hair passes through the keratinization transition portion existing below the hair bulb and sebaceous gland, moisture gradually escapes, and thus the hair changes into an oval shape and is stabilized due to the formation of cysteine bonds.

The hair cycle includes the anagen phase in which hair grows from stem cells, the catagen phase in which blood vessels fall off, and the telogen phase in which hair growth stops and hair sheds occurs because nutrient supply is completely stopped.

Reasons for alopecia, in which hair looks empty, may be various and include hormonal, immune, aging, and genetic factors, but the bottom line is that multiple hair follicles fail to transition into the anagen phase and stop in the telogen phase.

It was discovered in 2007 that stopped hair follicles can be activated again and transition into the growth phase through Wnt signaling (Gerge Cotsarelis et al., Nature. 2007 May 17;447(7142):316-20. doi: 10.1038/nature05766.).

Since then, in 2015, Dr. Christiano's research team at Columbia University demonstrated the hair growth efficacy with an already commercialized JAK inhibitor in animal and human tests (Harel et al. Sci. Adv. 2015;1:e1500973 23 October 2015).

Since then, the hair growth efficacy has been proven through various clinical studies. Successful results have also been reported for alopecia areata, alopecia areata with psoriasis, and androgenetic alopecia, which is not an autoimmune disease.

The JAK inhibition mechanism has been clearly scientifically proven to be a hair growth mechanism, and the JAK inhibition mechanism induces hair growth by stably inducing Wnt signaling.

In other words, this type of hair loss refers to a phenomenon in which the proportion of hair in the anagen phase in the hair growth cycle becomes smaller, the proportion of hair in the catagen or telogen phase increases, resulting in an abnormal increase in hair shedding, and hair that was growing normally falls out due to some cause. Although this hair loss is not directly related to life, it is often accompanied by serious mental pain in terms of appearance, and thus severe hair loss can have a very negative impact on the quality of life (Passchier J. et al., Dermatology, 197:217, 1998; McDonagh, A.J. and Messenger, A.G., Dermatol Clin., 14:661, 1996).

Damaged hair roots are also a problem, but in most cases of hair loss that is not hair loss caused by stress or disease, the hair naturally becomes thinner, weaker, and falls out as it loses nutrients. As this cycle accelerates, hair loss occurs in which the hair does not regenerate. In this process, not only the mechanism of action caused by male hormones, but also hair loss-inducing substances created by various factors such as stress, environmental pollution, irregular eating habits, and genetic factors, affect the Wnt signaling pathway and degrade beta-catenin. This induces cell apoptosis of dermal papilla cells and keratinocyte cells, thereby shortening the anagen phase of hair growth, resulting in transition from the anagen phase to the catagen phase, causing hair loss.

Recently, alopecia, which was considered a genetic disease in men, has appeared not only in men but also in women due to the above-described external factors, and thus the demand for drugs for hair loss prevention or treatment has increased.

Currently, hair growth-promoting agents such as minoxidil (agent for transdermal application) and finasteride (agent for oral administration) have received FDA approval and are used clinically. In particular, minoxidil was first introduced as a vasodilator in the early 1970s, and is currently used as a hair growth promoter since it was discovered that a side effect other than the drug effect was hair growth. Although mechanism of action of minoxidil has not been clearly elucidated to date, several studies suggested that the promotion of nutrient supply through vasodilation and the potassium channel opening effect induce hair growth.

However, hair growth drugs that are currently mainly used have been reported to show adverse reactions (minoxidil - weight gain, edema, increased heart rate, angina pectoris, dermatitis, itching, etc.), which significantly limit the use thereof in active treatment. Considering these problems, there is an urgent need to develop safe and highly effective natural raw materials that consumers can trust and use, and various studies thereon are being conducted.

Accordingly, the present inventors have discovered natural substances capable of reducing the side effects of conventional compositions for hair loss prevention and treatment compositions and replacing the same, and aim to provide a functional composition for hair loss for preventing and treating hair loss that acts effectively on not only male alopecia but also female alopecia by acting on the JAK-STAT signaling pathway and the Wnt signaling pathway.

### DISCLOSURE

### Technical Problem

An object of the present invention is to provide a composition for preventing and treating hair loss containing: a functional ingredient for hair loss extracted from a natural substance; a diluent; a surfactant; a co-surfactant; a moisturizer; and an emulsifier.

However, objects to be achieved by the present invention are not limited to the object mentioned above, and other objects not mentioned above can be clearly understood by those skilled in the art from the following description.

### Technical Solution

According to one embodiment of the present invention, there is provided a functional composition for hair loss containing: a functional ingredient for hair loss; a diluent; a surfactant; a co-surfactant; a moisturizer; and an emulsifier.

According to one embodiment of the present invention, the functional composition for hair loss may contain 5 to 50 wt% of the functional ingredient for hair loss, 10 to 30 wt% of the diluent, 20 to 50 wt% of the surfactant, 10 to 20 wt% of the co-surfactant, and 0.1 to 2 wt% of the emulsifier.

In addition, in the present invention, the functional ingredient for hair loss may comprise, as an active ingredient, any one or more selected from the group consisting of Brevilin A, Arnicolide D, Arnicolide C, and Microhelenin C.

In addition, in the present invention, the functional ingredient for hair loss may comprise, based on the total weight of the functional ingredient for hair loss, 10 to 90 wt% of Brevilin A, 0 to 35 wt% of Arnicolide D, 0 to 15 wt% of Arnicolide C, and 0 to 15 wt% of Microhelenin C.

In addition, in the present invention, the diluent may comprise any one or more selected from the group consisting of medium-chain triglyceride (MCT) oil , BRIJ 010-SS-(RB), Capryol 90, Capryol PGMC, Gantrez ES-435, Gantrez S-97 BF, Gelucire 43/01, Gelucire 44/14, Gelucire 50/13, Gelucire 48/16, Labrafac CC, Labrafac Lipophile WL1349, Labrafac PG, Labrafil M 1944 CS, Labrafil M 2125 CS, Labrafil M 2130 CS, Lauroglycol 90, Lauroglycol FCC, Monosteol, Peceol, Pharmasolve, Plurol Oleique CC497, Span 20-LQ-(SG), Span 60-PA-(SG), upper Refined Oleic Acid-LQ-(JP), Super Refined Tween 80A-LQ-(MH), Surfadone LP-300, TWEEN 60-SS-(SG), TWEEN 80 HP-LQ-(MH), TWEEN 80-LQ-(SG), and Vitamin E TPGS.

In addition, in the present invention, the surfactant may comprise any one or more selected from the group consisting of Labrasol, Crodex A-PA-(RB), Geleol, Gelot 64, Suppocire AS2X, Suppocire BML, Synperonic PE/F 127, Synperonic PE/L 44, and Tefose 63.

In addition, in the present invention, the co-surfactant may comprise any one or more selected from the group consisting of Transcutol, and Cremophor ELP.

In addition, in the present invention, the moisturizer may comprise a polyol comprising any one or more selected from the group consisting of D-panthenol, L-panthenol, panthenoic acid, glycerin, butylene glycol, propylene glycol, polyethylene glycol, and sorbitol.

In addition, in the present invention, the emulsifier may comprise a soybean-derived natural phospholipid comprising any one or more selected from the group consisting of lecithin, hydrogenated lecithin, unsaturated lecithin, and lysolecithin.

According to one embodiment of the present invention, there may be provided a cosmetic composition for preventing hair loss and promoting hair growth comprising the functional composition for hair loss.

According to one embodiment of the present invention, there may be provided an agent for preventing and treating hair loss comprising the functional composition for hair loss.

### Advantageous Effects

Embodiments of the present invention relate to a functional composition for hair loss, which contains a natural extract, can not only ensure the safety of the raw materials, but also ensure safety because of having no side effects compared to conventional hair growth agents, and exhibits better effects on hair loss prevention and treatment and hair growth promotion.

However, the effects of the present invention are not limited to the above-described effects, and should be understood to include all effects that can be inferred from the configuration of the invention described in the detailed description or the claims.

### Brief Description of Drawings

FIGS. 1 and 2 are images showing the anti-hair loss effects of Example 9 and Comparative Example 3 according to Experimental Example 1 of the present invention.
FIG. 3 is a graph showing the skin penetration rates of Examples and Comparative Examples according to Experimental Example 1 of the present invention
FIGS. 4 and 5 are graphs showing the NO production and cell viability of Brevilin A according to Experimental Example 2 of the present invention.
FIGS. 6 and 7 are graphs showing the NO production and cell viability of Arnicolide D according to Experimental Example 2 of the present invention.
FIGS. 8 and 9 are graphs showing the NO production and cell viability of Arnicolide C according to Experimental Example 2 of the present invention.
FIGS. 10 and 11 are graphs showing the NO production and cell viability of Microhelenin C according to Experimental Example 2 of the present invention.
FIG. 12 is a graph showing, as potency values, the inflammation inhibition rates of Brevilin A, Arnicolide D, Arnicolide C, and Microhelenin C according to Experimental Example 2 of the present invention.
FIGS. 13 and 14 are graphs showing the NO production and cell viability of CMX according to Experimental Example 2 of the present invention.
FIGS. 15 and 16 are graphs showing the NO production and cell viability of dexamethasone according to Experimental Example 2 of the present invention.

### Best Mode

According to one embodiment of the present invention, there is provided a functional composition for hair loss containing: a functional ingredient for hair loss; a diluent; a surfactant; a co-surfactant; a moisturizer; and an emulsifier.

### Mode for Invention

Hereinafter, a functional composition for hair loss and a preparation process therefor according to embodiments of the present invention will be described.

Prior to that, technical terms used in this specification is only for referring to specific embodiments and are not intended to limit the present invention. In addition, singular forms used herein also include plural forms, unless the phrases clearly indicate the opposite. In addition, the meaning of "comprising" or "containing" specifies a specific characteristic, region, integer, step, operation, element, or component, and is not intended to exclude the addition of other specific characteristics, regions, integers, steps, operations, elements, or components.

In the present invention, terms such as "first" and "second" are used to describe various components, and the terms are used only for the purpose of distinguishing one component from another component.

In addition, terms used in this specification are only used to describe exemplary embodiments, and are not intended to limit the present invention. Singular expressions include plural expressions, unless the context clearly dictates otherwise. It is to be understood that terms such as "comprise", "include", or "have", when used in the present invention, specify the presence of stated features, numbers, steps, components, or combinations thereof, but do not preclude the presence or existence of one or more other features, numbers, steps, components, or combinations thereof

The present invention can be modified variously and can have various forms, and thus specific embodiments will be illustrated and described in detail below. However, these embodiments are not intended to limit the present invention to a specific disclosed form, and should be understood to include all changes, equivalents, and substitutes included in the spirit and technical scope of the present invention. Hereinafter, the present invention will be described in more detail.

A functional composition for hair loss according to one embodiment of the present invention contains: a functional ingredient for hair loss; a diluent; a surfactant; a co-surfactant; a moisturizer; and an emulsifier.

Here, the hair loss may be an autoimmune disease. The autoimmune disease is a disease caused by an abnormal immune response due to autoimmunity, and examples thereof include, but are not limited to, hair loss, alopecia areata, rheumatoid arthritis, psoriasis, atopy, eczema, seborrheic dermatitis, scleroderma, skin immune hypersensitivity, multiple sclerosis, lupus, Behçet's disease, ankylosing spondylitis, Sjögren's syndrome, Crohn's disease, and inflammatory bowel disease.

The functional ingredient for hair loss comprises an extract from a natural substance, and may preferably comprise an extract from *Centipeda minima.*

In the present invention, the term "extracted substance" or "extract" includes an extract itself or an extract in any form that may be formed using the extract, such as an extract obtained by extraction treatment, a dilution or concentrate of the extract, a dried product obtained by drying the extract, a crude or purified product of the extract, or mixtures thereof.

In the present invention, drying of the extract may be performed by a known method as long as useful components from the collected natural substance are not destroyed. For example, the drying may be performed by natural drying in the shade. In addition, crushing or pulverization may be performed to form powder to the extent that useful components of the plant can be sufficiently extracted during the subsequent extraction process. The drying process and the crushing or pulverizing process can be performed in reverse order or repeatedly as needed.

For extraction in the present invention, the extraction method is not particularly limited, and extraction may be performed according to a method commonly used in the art. Non-limiting examples of the extraction method include hot water extraction, ultrasonic extraction, filtration, and reflux extraction, which may be performed alone or in combination of two or more.

The type of extraction solvent used to extract a natural substance in the present invention is not particularly limited, and any solvent known in the art may be used. In the present invention, the extract may be obtained by extraction with water, a C₁ to C₄ lower alcohol, hexane, or a mixed solvent thereof. In addition, non-limiting examples of the extraction solvent include water; C₁ to C₄ lower alcohols such as methanol, ethanol, propyl alcohol, and butyl alcohol; polyhydric alcohols such as glycerin, butylene glycol, and propylene glycol; and hydrocarbon solvents such as methyl acetate, ethyl acetate, acetone, benzene, hexane, diethyl ether, and dichloromethane; or mixtures thereof. Specifically, water, lower alcohol, 1,3-butylene glycol, ethyl acetate, and hexane may be used alone or in combination of two or more.

The term "compound" as used in the present invention refers to a result obtained by performing fractionation to separate a specific component or a specific component group from a mixture containing various components.

In the present invention, the separation method for obtaining the compound is not particularly limited and may be performed according to a method commonly used in the art. Non-limiting examples of the separation method include a method of obtaining a compound from a natural substance extract by treating the extract with a predetermined solvent.

The functional ingredient for hair loss according to the present invention may be contained in an amount of 5 to 50 wt% based on the total weight of the functional composition for hair loss. In order to ensure that the functional ingredient for hair loss is contained in an appropriate amount in the composition and provides excellent hair loss prevention and treatment, the functional ingredient for hair loss may preferably be contained in an amount of 5 to 50 wt%, without being limited thereto. In the present invention, if the content of the functional ingredient for hair loss is less than 5 wt% or more than 50 wt%, problems arise in that the amount of the component most related to the JAK-STAT signaling pathway is small, resulting in reduction in the anti-inflammatory effect, and activation of the Wnt (Wnt/β-catenin pathway) signaling pathway is inhibited due to the decrease in the synergy of the component with other components, making it difficult to sufficiently prevent and treat autoimmune diseases and hair loss.

In the present invention, the diluent, the surfactant, the co-surfactant, and the emulsifier may be contained in amounts of 10 to 35 wt%, 17 to 53 wt%, 7 to 25 wt%, and 0.1 to 3 wt%, respectively. In terms of ensuring that the functional ingredient for hair loss exhibits hair loss prevention, hair growth promotion, and anti-inflammatory effects, the diluent, the surfactant, the co-surfactant, and the emulsifier may preferably be contained in amounts of 10 to 30 wt%, 20 to 50 wt%, 10 to 20 wt%, and 0.1 to 2 wt%, respectively, without being limited thereto.

The functional ingredient for hair loss may comprise, as an active ingredient, any one or more selected from the group consisting of Brevilin A, Arnicolide D, Arnicolide C, and Microhelenin C.

Brevilin A is represented by the following formula:

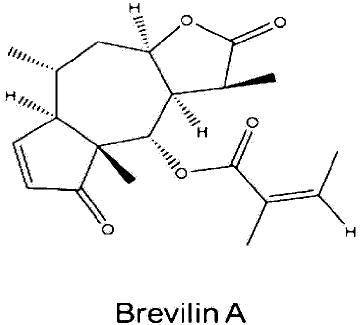

Brevilin A has CAS number 16503-32-5, a molecular formula of C₂₀H₂₆O₅, and a molecular weight of 346.42, and is derived from *Arnica* sp., *Centipeda* sp., or *Helenium* sp.

A number of documents indicating that Brevilin A is contained in *Centipeda minima* have been found, and some documents reported that Brevilin A is also contained in *Litsea glutinous.* In addition, documents indicating that a certain amount of Brevilin A is also contained in plants of the genus *Arnica (Arnica longifolia, Arctium lappa, Arnica chamissonis subsp. Foliosa,* and *Arnica chamissonis Less.*) and plants of the genus *Helenium* (*Helenium autumnale L, Helenium alternifolium, Helenium pinnatifidum, Helenium vernale, Helenium brevifolium,* and *Helenium flexuosum*) have been found

Arnicolide D is represented by the following formula:

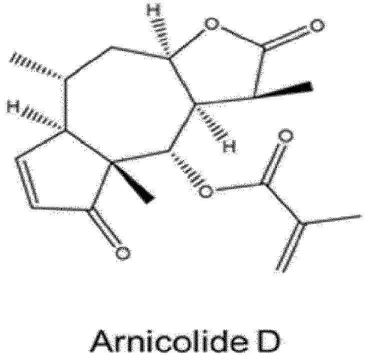

Arnicolide D has CAS number 34532-68-8, a molecular formula of C₁₉H₂₄O₅, and a molecular weight of 332.40, and is derived from *Arnica* sp., or *Centipeda* sp.

Arnicolide C is represented by the following formula:

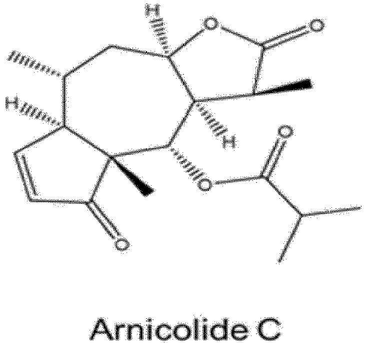

Arnicolide C has CAS number 34532-68-7, a molecular weight of C₁₆H₂₆O₅, and a molecular weight of 334.41, and is derived from *Arnica* sp., or *Centipeda* sp.

Microhelenin C is represented by the following formula:

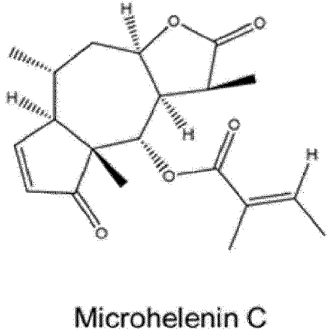

Microhelenin C has CAS number 63569-07-3, a molecular formula of C₂₀H₂₆O₅, and a molecular weight of 346.4, and is derived from *Centipeda* sp., or *Helenium* sp.

The mechanism of action of each of Brevilin A, Arnicolide D, Arnicolide C, and Microhelenin C is as follows.

Brevilin A suppresses or inhibits the JAK-STAT signaling pathway.

Brevilin A, a natural substance, is widely known to inhibit JAK (Janus kinase activity) and STAT3 signaling in cancer cells (PLoS One 8 (5) (2013) e63697). In addition, it is effective in treating alopecia areata, a type of autoimmune disease, by suppressing strong inflammatory responses and various inflammation-related targets through inhibition of the JAK-STAT (signal transducers and activators of transcription) pathway.

Arnicolide D, Arnicolide C and Microhelenin C inhibit or suppress cytokine-cytokine receptor interactions, the MAPK (mitogen-activated protein kinase) signaling pathway, and the PI3K-AKT signaling pathway (PI3K (phosphatidylinositol-3-kinase)-AKT signaling pathway).

Brevilin A, Arnicolide D, Arnicolide C and Microhelenin C activate the Wnt (Wnt/β-catenin pathway) signaling pathway, thereby promoting the differentiation and proliferation of hair follicle stem cells and primary hair cells involved in hair growth.

Brevilin A is the most important component for anti-autoimmune diseases, such as hair growth, anti-hair loss, and anti-inflammation, and the content thereof is preferably higher than the total content of other derivatives, Arnicolide D, Arnicolide C, and Microhelenin C. The major component of the non-polar natural substance of the raw material *Centipeda minima* is Brevilin A.

Brevilin A is most related to the JAK-STAT signaling pathway, which is the main mechanism of action in autoimmune diseases, and when it acts together with other derivatives, Arnicolide D, Arnicolide C, and Microhelenin C, they exhibit a synergistic effect against autoimmune diseases. Thus, using these components in combination is more effective in preventing and treating autoimmune diseases, such as hair loss, than when each component is used alone.

In the present invention, Brevilin A is contained in an amount of 10 to 90 wt% based on the total weight of the functional ingredient for hair loss. If the content of Brevilin A is less than 10 wt% or more than 90 wt%, problems arise in that the amount of the component most related to the JAK-STAT signaling pathway is small, resulting in reduction in the anti-inflammatory effect or reduction in the synergy of the component with other components, making it difficult to sufficiently prevent and treat autoimmune diseases. However, the present invention is not limited thereto.

For example, the potency calculation formula for Brevilin A may be y = 0.9422×1² - 8.7333x1 + 20.889. Here, x1 is the concentration (µM) of Brevilin A, and y is the concentration (µM) of NO.

The NO concentration on the y-axis to demonstrate effective anti-inflammatory efficacy is 10 µM or less, preferably 9 µM or less, more preferably 8 µM or less. This is preferably applied not only to the concentration of Brevilin A, but also to the potency calculation formulas for Arnicolide D, Arnicolide C, and Microhelenin C below.

In the present invention, Arnicolide D may be contained in an amount of 0 to 35 wt%, 5 to 34 wt%, or 10 to 30 wt%, based on the total weight of the functional ingredient for hair loss. In order to sufficiently prevent and treat autoimmune disease and hair loss through a synergistic effect with other ingredients such as Brevilin A, Arnicolide C, and Microhelenin C, Arnicolide D may preferably be contained in an amount of 15 to 30 wt%, without being limited thereto.

For example, the potency calculation formula for Arnicolide D may be y = 0.3259×2² - 3.4444x2 + 9.8519, and an effect of 1.5 relative to the same amount of Brevilin A may appear. Here, x2 is the concentration (µM) of Arnicolide D, and y is the concentration (µM) of NO.

In the present invention, Arnicolide C may be contained in an amount of 0 to 15 wt%, 0.1 to 14 wt%, 0.5 to 13 wt%, or 1 to 10 wt%, based on the total weight of the functional ingredient for hair loss. In order to sufficiently prevent and treat autoimmune disease and hair loss through a synergistic effect with the other components Brevilin A, Arnicolide D, and Microhelenin C, Arnicolide C may preferably be contained in an amount of 1 to 8 wt%, without being limited thereto.

For example, the potency calculation formula for Arnicolide C may be y = 0.3378×3² - 4.8222x3 + 18, and an effect of 0.7 relative to the same amount of Brevilin A may appear. Here, x3 is the concentration (µM) of Arnicolide C, and y is the concentration (µM) of NO.

In the present invention, Microhelenin C may be contained in an amount of 0 to 15 wt%, 0.1 to 14 wt%, 0.5 to 13 wt%, or 1 to 10 wt%, based on the total weight of the functional ingredient for hair loss. In order to sufficiently prevent and treat autoimmune disease and hair loss through a synergistic effect with the other components Brevilin A, Arnicolide D, and Arnicolide C, Microhelenin C may preferably be contained in an amount of 1 to 8 wt%, without being limited thereto.

For example, the potency calculation formula for Microhelenin C may be y = 0.2726×4² - 4.3111x4 + 16.13, and an effect of 0.8 relative to the same amount of Brevilin A may appear. Here, x4 is the concentration (µM) of Microhelenin C, and y is the concentration (µM) of NO.

Therefore, if the content of each of Brevilin A, Arnicolide D, Arnicolide C, and Microhelenin C is out of the above range, the desired anti-hair loss and anti-inflammatory effects cannot be achieved.

In the present invention, the diluent may comprise any one or more selected from the group consisting of medium-chain triglyceride (MCT) oil, BRIJ 010-SS-(RB), Capryol 90, Capryol PGMC, Gantrez ES-435, Gantrez S-97 BF, Gelucire 43/01, Gelucire 44/14, Gelucire 50/13, Gelucire 48/16, Labrafac CC, Labrafac Lipophile WL1349, Labrafac PG, Labrafil M 1944 CS, Labrafil M 2125 CS, Labrafil M 2130 CS, Lauroglycol 90, Lauroglycol FCC, Monosteol, Peceol, Pharmasolve, Plurol Oleique CC497, Span 20-LQ-(SG), Span 60-PA-(SG), upper Refined Oleic Acid-LQ-(JP), Super Refined Tween 80A-LQ-(MH), Surfadone LP-300, TWEEN 60-SS-(SG), TWEEN 80 HP-LQ-(MH), TWEEN 80-LQ-(SG), and Vitamin E TPGS.

In the present invention, the surfactant may comprise any one or more selected from the group consisting of Labrasol, Crodex A-PA-(RB), Geleol, Gelot 64, Suppocire AS2X, Suppocire BML, Synperonic PE/F 127, Synperonic PE/L 44, and Tefose 63.

In the present invention, the co-surfactant may comprise any one or more selected from the group consisting of Transcutol, and Cremophor ELP.

In present invention, the moisturizer may comprise a polyol comprising any one or more selected from the group consisting of D-panthenol, L-panthenol, panthenoic acid, glycerin, butylene glycol, propylene glycol, polyethylene glycol, and sorbitol.

In the present invention, the emulsifier may comprise a soybean-derived natural phospholipid comprising any one or more selected from the group consisting of lecithin, hydrogenated lecithin, unsaturated lecithin, and lysolecithin.

According to one embodiment of the present invention, there may be provided a cosmetic composition for preventing hair loss and promoting hair growth comprising the functional composition for hair loss.

In this specification, the term "cosmetic composition" refers to a product that is used on the human body in order to clean and beautify the human body to add attractiveness, brighten the appearance, or maintain or promote the skin and hair health. The cosmetic composition may be for antimicrobial, anti-inflammatory, and hair loss prevention and treatment purposes.

The cosmetic composition may be a cosmetic composition containing at least one selected from the group consisting of a thickener, a preservative, a stabilizer, a solubilizer, a surfactant, a carrier, and a fragrance. The cosmetic composition may also additionally contain other components and functional components that are added to conventional cosmetics.

Other components that may be added include oil components, moisturizers, emollients, surfactants, organic and inorganic pigments, organic powders, ultraviolet absorbers, preservatives, disinfectants, antioxidants, plant extracts, pH adjusters, alcohol, colorants, fragrances, blood circulation promoters, cooling agents, antiperspirants, purified water, etc.

The functional additives may include components selected from the group consisting of water-soluble vitamins, oil-soluble vitamins, high-molecular-weight peptides, high-molecular-weight polysaccharides, sphingolipids, and seaweed extracts, and may further include woody extract, *Houttuynia cordata* extract, rosemary extract, garlic extract, and yellow lotus extract.

Examples of the oil components include ester-based oils, hydrocarbon-based oils, silicone-based oils, fluorine-based oils, animal oils, vegetable oils, and the like.

Examples of the moisturizer include water-soluble low-molecular-weight moisturizers, oil-soluble molecular moisturizers, water-soluble polymers, and oil-soluble polymers.

Examples of the emollient include long-chain acyl glutamic acid cholesteryl ester, hydroxystearic acid cholesteryl, 12-hydroxystearic acid, stearic acid, rosin acid, lanolin fatty acid cholesteryl ester, and the like.

Examples of the organic and inorganic pigments include silicic acid, anhydrous silicic acid, magnesium silicate, talc, sericite, mica, kaolin, bengala, clay, bentonite, titanium-coated mica, bismuth oxychloride, zirconium oxide, magnesium oxide, zinc oxide, titanium oxide; inorganic pigments such as aluminum oxide, calcium sulfate, barium sulfate, magnesium sulfate, calcium carbonate, magnesium carbonate, iron oxide, ultramarine blue, chromium oxide, chromium hydroxide, calamine, and complexes thereof; polyamide, polyester, polypropylene, polystyrene, polyurethane, vinyl resin, urea resin, phenol resin, fluorine resin, silicon resin, acrylic resin, melamine resin, epoxy resin, polycarbonate resin, divinylbenzene/styrene copolymers, silk powder, cellulose, organic pigments such as CI pigment yellow and CI pigment orange, and complex pigments of these inorganic pigments and organic pigments.

Examples of the organic powder include metal soap such as calcium stearate; alkyl phosphate metal salts such as sodium zinc cetylate, zinc laurylate, and calcium laurylate; acyl amino acid polyvalent metal salts such as N-lauroyl-beta-alanine calcium, N-lauroyl-beta-alanine zinc, and N-lauroyl glycine calcium; amidesulfonic acid polyvalent metal salts such as N-lauroyl-taurine calcium and N-palmitoyl-taurine calcium; N-acyl basic amino acids such as N-epsilon-lauroyl-L-lysine, N-epsilon-palmitoyl-lysine, N-alpha-palmitoyl ornithine, N-alpha-lauroyl arginine, and N-alpha-hydrogenated beef fatty acid acylarginine; N-acyl polypeptides such as N-lauroylglycylglycine; alpha-amino fatty acids such as alpha-aminocaprylic acid and alpha-aminolauric acid; polyethylene, polypropylene, nylon, polymethyl methacrylate, polystyrene, divinylbenzene/styrene copolymers, tetrafluoroethylene, and the like.

Examples of the ultraviolet absorber include para-aminobenzoic acid, ethyl para-aminobenzoate, amyl para-aminobenzoate, octyl para-aminobenzoate, ethylene glycol salicylate, phenyl salicylate, octyl salicylate, benzyl salicate, butylphenyl salicylate, homomenthyl salicylate, benzyl cinnamate, paramethoxycinnamic acid-2-ethoxyethyl, octyl para-methoxycinnamate, mono-2-ethylhexane glyceryl di-para-methoxycinnamate, isopropyl para-methoxycinnamate, a diisopropyl/diisopropyl cinnamic acid ester mixture, urocanic acid, ethyl urocanate, hydroxymethoxybenzophenone, hydroxymethoxybenzophenone sulfonic acid and salts thereof, dihydroxymethoxybenzophenone, dihydroxymethoxybenzophenone disulfonate sodium, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, etc.

Examples of the disinfectants include hinokitiol, triclosan, trichlorohydroxydiphenyl ether, chlorhexidine gluconate, phenoxyethanol, resorcin, isopropylmethylphenol, azulene, salicylic acid, zinc phyllithione, benzalkonium chloride, photosensitizer No. 301, sodium mono-nitroguaiacol, undecylenic acid, etc.

Examples of the antioxidant include butylhydroxyanisole, propyl gallate, and erythorbic acid.

Examples of the pH adjusting agent include citric acid, sodium citrate, malic acid, sodium malate, fumaric acid, sodium fumarate, succinic acid, sodium succinate, sodium hydroxide, sodium monohydrogen phosphate, and the like.

In addition, the components that may be added are not limited thereto, and any of the components may be added within the range that does not impair the purpose and effect of the cosmetic composition.

The formulation of the cosmetic composition is not particularly limited, but may preferably be selected from the group consisting of skin lotion, skin softener, skin toner, astringent, lotion, milk lotion, moisturizing lotion, nourishing lotion, massage cream, nourishing cream, moisturizing cream, hand cream, foundation, essence, nourishing essence, pack, soap, cleansing foam, cleansing lotion, cleansing cream, body lotion, and body cleanser, and may be any one formulation selected from the group consisting of skin external preparations, quasi-drugs, and pharmaceutical compositions for anti-inflammation or hair loss prevention and hair growth promotion.

The skin external preparation may be a cream, gel, ointment, skin emulsifier, skin suspension, transdermal delivery patch, drug-containing bandage, lotion, or a combination thereof. The skin external preparation may, if necessary, contain components commonly used in skin external preparations such as cosmetics and medicines, for example, aqueous components, oilbased components, powder components, alcohols, moisturizers, thickeners, ultraviolet absorbers, whitening agents, preservatives, antioxidants, surfactants, fragrances, colorants, various skin nutrients, or combinations thereof. The skin external preparation may also appropriately contain metal sequestrants such as disodium edetate, trisodium edetate, sodium citrate, sodium polyphosphate, sodium metaphosphate, and gluconic acid; caffeine, tannin, verapamil, licorice extract, glabridin, hot water extract of calines from fruit, various herbal medicines, medicines such as tocopherol acetate, glycyrrhizic acid, tranexamic acid, or a derivative or salt of any of the foregoing; vitamin C, magnesium ascorbate phosphate, ascorbic acid glucoside, arbutin, kojic acid; and sugars such as glucose, fructose, and trehalose.

The skin includes all skin areas of the body, including the face, hands, arms, legs, feet, chest, abdomen, back, buttocks, and scalp.

As used herein, the terms "applying" and "administering" are used interchangeably and may mean resulting in at least partial localization of the composition according to one embodiment of the present invention to a desired site or placing the composition according to one embodiment of the present invention into a subject by a route of administration.

According to one embodiment of the present invention, there may be provided an agent for preventing and treating hair loss comprising the functional composition for hair loss.

The present invention is provided based on the following experimental results, and the functional composition for hair loss according to the present invention is characterized by containing, as an active ingredient, any one or more selected from the group consisting of Brevilin A, Arnicolide D, Arnicolide C, and Microhelenin C. If the agent is in a form comprising the functional composition for hair loss, it may be usefully used in groups related to cosmetics, health functional foods, skin external preparations, quasi-drugs, and pharmaceutical compositions.

Hereinafter, the functional composition for hair loss according to the present invention will be described in more detail by way of examples.

### Examples

An extract from *Centipeda minima* was used as a functional ingredient for hair loss, and the extract contained the active ingredients Brevilin A, Arnicolide D, Arnicolide C, and Microhelenin C, and was named CMX.

The *Centipeda minima* extract used was an extract from domestically produced *Centipeda minima* (Yeongcheon, Korea) purchased from the Goesan Herbal Agricultural Products Cooperative (https://natural-herb.co.kr). Specifically, 4,320 g of dried Centipeda minima hay was precipitated in 40 L of 70% ethanol for 48 hours, subjected to maceration and extraction with the alcohol, and filtered to obtain 31.5 kg of primary extract. Then, 32 L of hexane was added to and mixed with the primary extract, and the mixture was left to stand for 2 hours to remove the hexane layer. After removing the hexane layer, 160 g of Labrafac as a lipophilic solubilizer was added to 31.5 kg of the remaining extract, and the mixture was stirred for 10 minutes, concentrated under reduced pressure to remove ethanol, and then left to stand for 60 minutes to remove the phase-separated supernatant.

### Experimental Example 1. Evaluation of Efficacy Depending on Content of Functional Ingredient for Hair Loss

### 1-1. Examples 1 to 19

**[Table 1]**

| Classification (wt%) | Example 1 | Example 2 | Example 3 | Example 4 | Example 5 | Example 6 | Example 7 | Example 8 | Example 9 |
|---|---|---|---|---|---|---|---|---|---|
| Functional ingredient for hair loss (CMX) | 5 | 10 | 10 | 20 | 30 | 40 | 49.8 | 37.5 | 37.5 |
| Emulsifier | 2 | 2 | 2 | 1 | 0.2 | 0.2 | 0.2 | 0.2 | 0.2 |
| Surfactant | 43 | 38 | 38 | 34 | 20 | 20 | 20 | 32.3 | 20 |
| Co-surfactant | 20 | 20 | 20 | 20 | 19.8 | 20 | 20 | 20 | 32.3 |
| Diluent | 30 | 30 | 30 | 25 | 30 | 19.8 | 10 | 10 | 10 |

**[Table 2]**

| Classification (wt%) | Example 10 | Example 11 | Example 12 | Example 13 | Example 14 | Example 15 | Example 16 | Example 17 | Example 18 | Example 19 |
|---|---|---|---|---|---|---|---|---|---|---|
| Functional ingredient for hair loss (CMX) | 40 | 50 | 37.5 | 30 | 40 | 49.8 | 49.8 | 47.5 | 47.5 | 47.5 |
| Emulsifier | 0.2 | 0.2 | 0.2 | 0.2 | 0.2 | 0.2 | 0.2 | 0.2 | 0.2 | 0.2 |
| Surfactant | 29.8 | 20 | 32.3 | 20 | 29.8 | 20 | 20 | 20 | 20 | 20 |
| Co-surfactant | Transcut ol | Gelucire 44/14 | Labrafil M 1944 CS | Capryol PGMC | Labrafil M 2130 CS | Labrafil M 2125 CS | Pharmas olve | TWEEN 60-SS-(SG) | Vitamin E TPGS | Transcut ol |
| | 10 | 19.8 | 20 | 19.8 | 20 | 20 | 20 | 19.8 | 19.8 | 19.8 |
| Diluent | MCT oil | Gelucire 43/01 | Capryol 90 | Laurogl ycol 90 | Monoste ol | Peceol | Plurol Oleique CC497 | Span 60-PA-(SG) | Capryol PGMC | MCT oil |
| | 10 | 10 | 10 | 30 | 10 | 10 | 10 | 12.5 | 12.5 | 12.5 |

As shown in Tables 1 and 2 above, Examples 1 to 19 contained the functional ingredient for hair loss (CMX) according to the present invention in an amount of 5 to 50 wt% and contained lecithin as an emulsifier and Labrasol as a surfactant. Examples 1 to 9 contained Transcutol as a co-surfactant and Labrafac as a diluent, and Examples 10 to 19 included different types of co-surfactants and diluents. In addition, the Examples contained 0.1 to 2 wt% of the emulsifier, 20 to 50 wt% of the surfactant, 10 to 20 wt% of the co-surfactant, and 10 to 30 wt% of the diluent.

The results of evaluating the efficacies of the compositions according to Tables 1 and 2 above are shown in Tables 3 and 4 below. Specifically, the formulations were evaluated whether layer separation occurred, whether skin toxicity occurred, their anti-hair-loss effect, and their skin penetration rate.

On 38 male and female test subjects (aged 30 to 60 years) diagnosed with androgenetic alopecia, whether layer separation occurred, whether skin toxicity occurred, and the anti-hair-loss effect were evaluated through the experiment conducted for 24 weeks. Specifically, each test subject did not use any special hair products or hair care or manipulation during the study period, and self-applied 0.5 mL of each sample once to the skin after shampooing every evening and sufficiently drying the hair. After the 24-week experiment was completed, whether skin toxicity occurred and the anti-hair-loss effect were evaluated, and whether layer separation in the composition occurred was visually evaluated. (The anti-hair loss effect was evaluated by the test subjects and rated as follows: O: effective in alleviating hair loss, Δ: little effective in improving hair loss, and X: not effective at all in alleviating hair loss).

Next, the experiment on skin penetration rate was conducted using the Franz diffusion cell method using the skins of hairless mice. Specifically, 105 µg/cm² of each composition was used, and 4 hours after application, drug release was measured and evaluated by HPLC at 365 nm using 0.5 mL of pH 7.4 PBS buffer.

**[Table 3]**

| Classification | Example 1 | Example 2 | Example 3 | Example 4 | Example 5 | Example 6 | Example 7 | Example 8 | Example 9 |
|---|---|---|---|---|---|---|---|---|---|
| Layer separation | Not occurred | Not occurred | Not occurred | Not occurred | Not occurred | Not occurred | Not occurred | Not occurred | Not occurred |
| Skin toxicity | Not irritating | Not irritating | Not irritating | Not irritating | Not irritating | Not irritating | Not irritating | Not irritating | Not irritating |
| Anti-hair-loos effect | O | O | O | O | O | O | O | O | O |
| Skin penetration rate (%) | 105.12 | 107.38 | 107.29 | 112.23 | 121.83 | 165.19 | 184.54 | 146.71 | 181.54 |

**[Table 4]**

| Classification | Example 10 | Example 11 | Example 12 | Example 13 | Example 14 | Example 15 | Example 16 | Example 17 | Example 18 | Example 19 |
|---|---|---|---|---|---|---|---|---|---|---|
| Layer separation | Not occurred | Not occurred | Not occurred | Not occurred | Not occurred | Not occurred | Not occurred | Not occurred | Not occurred | Not occurred |
| Skin toxicity | Not irritating | Not irritating | Not irritating | Not irritating | Not irritating | Not irritating | Not irritating | Not irritating | Not irritating | Not irritating |
| Anti-hair-loos effect | O | O | O | O | O | O | O | O | O | O |
| Skin penetration rate (%) | 158.74 | 184.69 | 146.68 | 122.01 | 157.89 | 184.5 | 184.61 | 172.57 | 173.11 | 172.28 |

As shown in Tables 3 and 4 above, it could be confirmed, in all of Examples 1 to 19, which contained the functional ingredient for hair loss (CMX) in an amount ranging from 5 to 50 wt%, layer separation and skin toxic reaction did not occur, and the anti-hair-loss effect was exhibited, and that they all exhibited a skin penetration rate of 100% or more. In addition, it could be confirmed that, when the composition contained the CMX in an amount of 5 to 50 wt%, it exhibited excellent efficacy regardless of the contents (wt%) of the emulsifier and surfactant or the types and contents (wt%) of co-surfactant and diluent.

### 1-2. Comparative Examples 1 to 6

**[Table 5]**

| Classification (wt%) | Comparative Example 1 | Comparative Example 2 | Comparative Example 3 | Comparative Example 4 | Comparative Example 5 | Comparative Example 6 |
|---|---|---|---|---|---|---|
| Functional ingredient for hair loss (CMX) | 55 | 59.9 | 0 | 0 | 1 | 3 |
| Emulsifier | 0.1 | 0.1 | 0.1 | 0 | 0.2 | 0.2 |
| Surfactant | 20 | 20 | 20 | 0 | 50 | 50 |
| Co-surfactant | 14.9 | 10 | 15 | 52.5 | 18.8 | 16.8 |
| Diluent | 10 | 10 | 59.9 | 47.5 | 30 | 30 |

As shown in Table 5 above, Comparative Examples 1 to 6 contained the functional ingredient for hair loss (CMX) according to the present invention in an amount out of the content range of 5 to 50 wt%, and contained lecithin as an emulsifier, Labrasol as a surfactant, Transcutol as a co-surfactant, and Labrafac as a diluent.

The results of evaluating the efficacies of the compositions according to Table 5 above are shown in Table 6 below. On 12 male and female test subjects (aged 30 to 60 years) diagnosed with androgenetic alopecia, whether layer separation of the composition occurred, whether skin toxicity occurred, the anti-hair-loss effect, and the skin penetration rate were evaluated in the same manner as in Example 1-1 above.

**[Table 6]**

| | Comparative Example 1 | Comparative Example 2 | Comparative Example 3 | Comparative Example 4 | Comparative Example 5 | Comparative Example 6 |
|---|---|---|---|---|---|---|
| Layer separation | Not occurred | Not occurred | Not occurred | Not occurred | Occurred | Occurred |
| Skin toxicity | Irritating | Irritating | Not irritating | Not irritating | Not irritating | Not irritating |
| Anti-hair-loss effect | O | O | X | X | Δ | Δ |
| Skin penetration rate | 183.12 | 184.93 | 0 | 0 | 10.95 | 18.91 |

As shown in Table 6 above, if the content of the functional ingredient for hair loss (CMX) according to the present invention is out of the range specified in the present invention, a problem may arise in that layer separation of the formulation or skin toxicity occurs or the skin penetration rate is significantly low.

Specifically, it could be seen that, in the case of Comparative Examples 1 and 2 containing more than 50 wt% of the functional ingredient for hair loss (CMX), the skin penetration rate was somewhat similar to that of Example 9, but skin irritation occurred, indicating that skin toxicity occurred.

It could be seen that, in the case of Comparative Examples 3 and 4 did not contain the functional ingredient for hair loss (CMX), there was no component that penetrated the skin, and thus no anti-hair-loss effect appeared.

It could be seen that, in the case of Comparative Examples 5 and 6 containing less than 5 wt% of the functional ingredient for hair loss (CMX), layer separation in the formulation occurred, the skin penetration rate was significantly low, and thus almost no anti-hair-loss effect appeared. In addition, it could be confirmed that, compared to Example 1 containing 5 wt% of the functional ingredient for hair loss (CMX), the skin penetration rate was significantly lower (different by 85% or more).

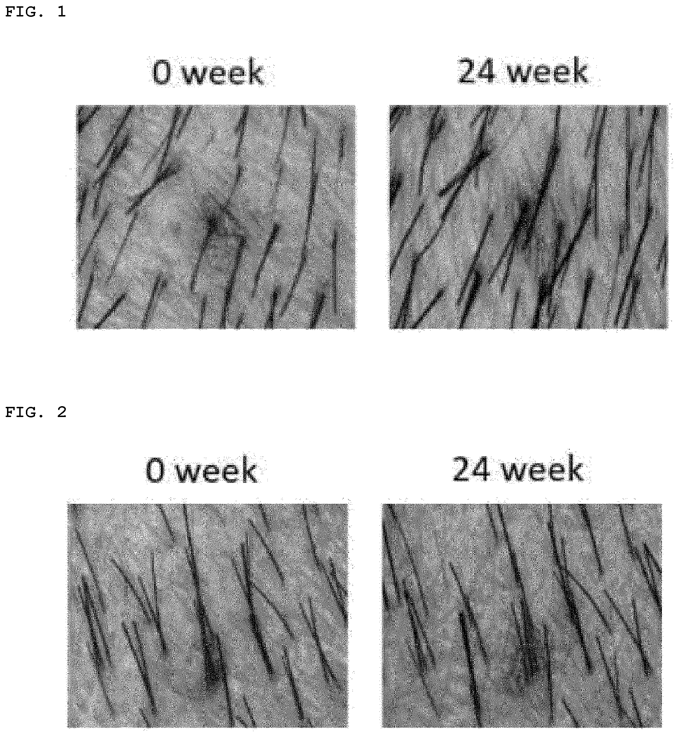

Next, referring to FIGS. 1 and 2, when comparing Example 9 containing the functional ingredient for hair loss (CMX) with Comparative Example 3 not containing the functional ingredient for hair loss (CMX), it could be confirmed that hair growth or anti-hair loss at week 24 was significantly improved in Example 9.

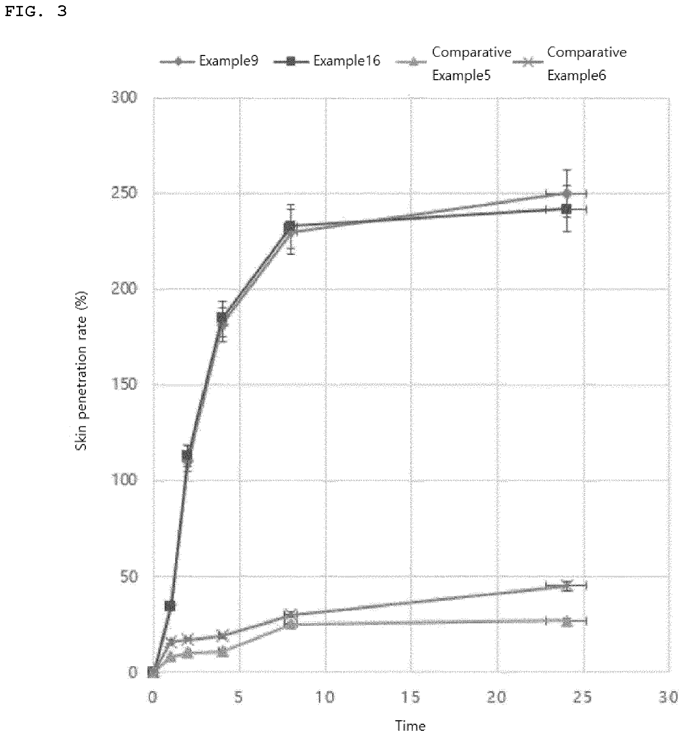

Referring to FIG. 3, it could be confirmed that, in the case of Examples 9 and 16, the skin penetration rate of the functional ingredient for hair loss (CMX) was significantly higher than that in Comparative Examples 5 and 6 containing less than 5 wt% of the functional ingredient for hair loss (CMX).

Accordingly, it could be seen that, when the functional ingredient for hair loss (CMX) according to the present invention was contained in an amount of 5 to 50 wt%, it was possible to prepare a stable formulation without causing layer separation, and the formulation was not irritating to the skin, had a high skin penetration rate, and was effective in alleviating hair loss.

### Experimental Example 2. Anti-Inflammatory Effect of Functional Ingredient for Hair Loss

In order to evaluate the anti-inflammatory effect of the functional composition for hair loss (CMX) according to the present invention, an NO production inhibition measurement experiment and a cell viability measurement experiment were conducted on each of Brevilin A, Arnicolide D, Arnicolide C, and Microhelenin C, which are the components of CMX, and on CMX containing all of the components. In addition, for comparison, an NO production inhibition measurement experiment and a cell viability measurement experiment were also conducted on the synthetic anti-inflammatory agent dexamethasone.

The experiments were conducted using mouse macrophage RAW 264.7 cells as an inflammatory model. RAW 264.7 cells were cultured in a 100 mm cell culture dish with DMEM containing 10% fetal bovine serum and 1% penicillin in a 5% CO₂ incubator. Cells that reached confluence were detached using a scraper and subcultured.

Specifically, for the NO production inhibition measurement experiment, which is an anti-inflammatory experiment, RAW 264.7 cells were dispensed on a 60-mm plate at a density of 2 × 10⁶ cells/dish and attached to the plate for 24 hours. Then, the cells were treated with 100 ng/ml of lipopolysaccharide (LPS) to induce an inflammatory response, and then treated with fresh media containing each component (whose anti-inflammatory effect was to be evaluated) at concentrations of 1.25, 2.5, 5, and 10 ug/ml, followed by 24 hours of culture. The amount of NO produced by the cultured RAW 264.7 cells and present in the culture medium was measured using a NO detection kit (cat. No. 21021, Intron) based on the Griess reaction. 100 µl of the culture medium estimated to contain NO was dispensed into 96-well plates, and then 50 µl of N1 buffer (sulfanilamide) was added thereto and reacted at room temperature for 10 minutes. Next, 50 µl of N2 buffer (naphthylethylenediamine) was added thereto and reacted at room temperature for 10 minutes, and then the absorbance was measured at 540 nm. The amount of NO produced was determined using a standard calibration curve obtained using a nitrite standard. In addition, a positive control group (CTL) was treated with only LPS and fresh medium not containing any of the components, and a negative control group was treated with only 100 ng/ml LPS. The measurement results are presented as the average value of three repeated experiments.

Specifically, for the experiment on cell viability, RAW 264.7 cells were dispensed on a 60-mm plate at a density of 2 × 10⁶ cells/dish, and attached to the plate for 24 hours. Then, the cells were treated with fresh media containing each component at concentrations of 1.25, 2.5, 5 and 10 ug/ml and cultured for 24 hours. The number of viable cells was counted by measuring the dehydrogenase present in the mitochondria within the cultured cells. EZ-CYTOX reagent (EZ-1000, Daeilab) was used, and based on the absorbance measured at 450 nm, the cell viability for each concentration was calculated according to the following equation: Cell viability = (absorbance of CMX-treated group/absorbance of untreated group) × 100. In addition, the measurement result is presented as the average value of three repeated experiments, and is a value measured relative to the positive control group (0 wt% of components).

### 2-1. Anti-inflammatory Effect and Cell Viability of Functional Ingredient for Hair Loss

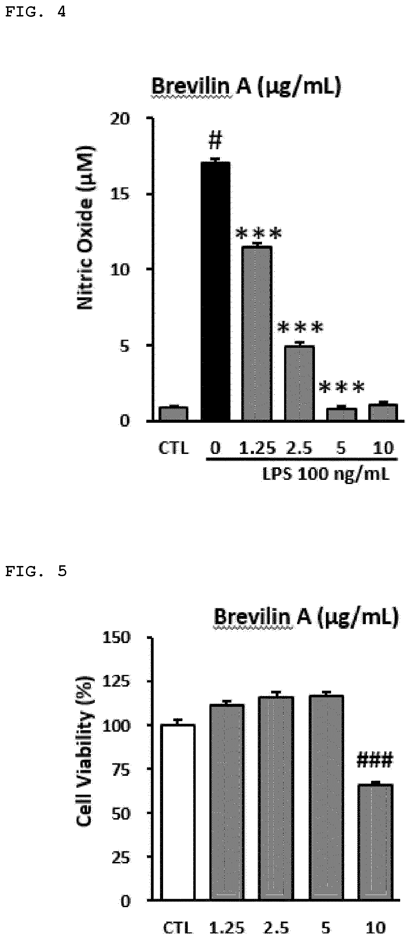

Referring to FIGS. 4 and 5, it could be seen that, as the concentration of Brevilin A increased, the overall NO production decreased, but there was no significant change in cell viability, indicating that Brevilin A did not cause cytotoxicity. Specifically, it could be confirmed that, when the concentration of Brevilin A was 5 pg/mL, the production of NO was the lowest and the cell viability was the highest, indicating that that Brevilin A at this concentration was not cytotoxic.

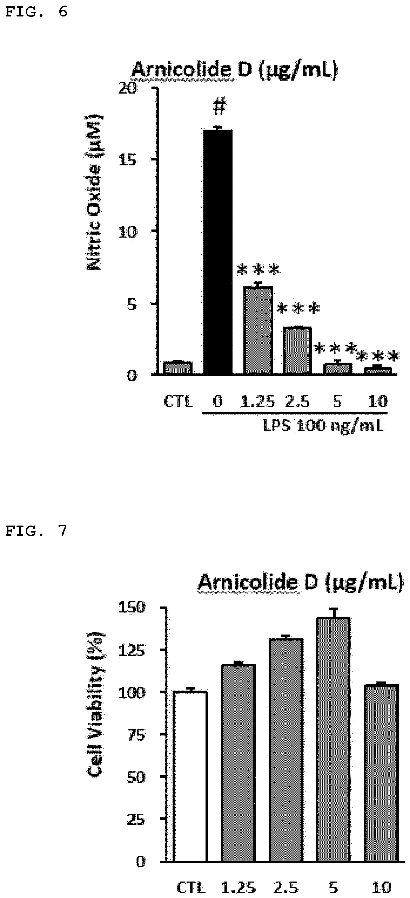

Referring to FIGS. 6 and 7, it could be seen that, as the concentration of Arnicolide D increased, the overall NO production decreased, but there was no significant change in cell viability, indicating that Arnicolide D did not cause cytotoxicity. Specifically, it could be confirmed that, when the concentration of Arnicolide D was 5 pg/mL, the cell viability was the highest while the production of NO was lower than that in the positive control group (CTL).

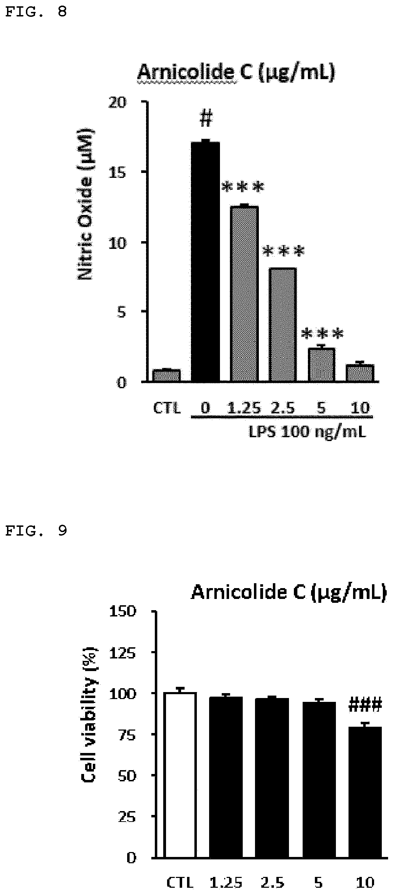

Referring to FIGS. 8 and 9, it could be seen that, as the concentration of Arnicolide C increased, the overall NO production decreased, but there was no significant change in cell viability, indicating that Arnicolide C did not cause cytotoxicity. Specifically, it could be confirmed that, when the concentration of Arnicolide C was 10 pg/mL, the production of NO was the lowest and the difference in cell viability from the positive control group (CTL) was significantly small, indicating that Arnicolide C at this concentration was not cytotoxic.

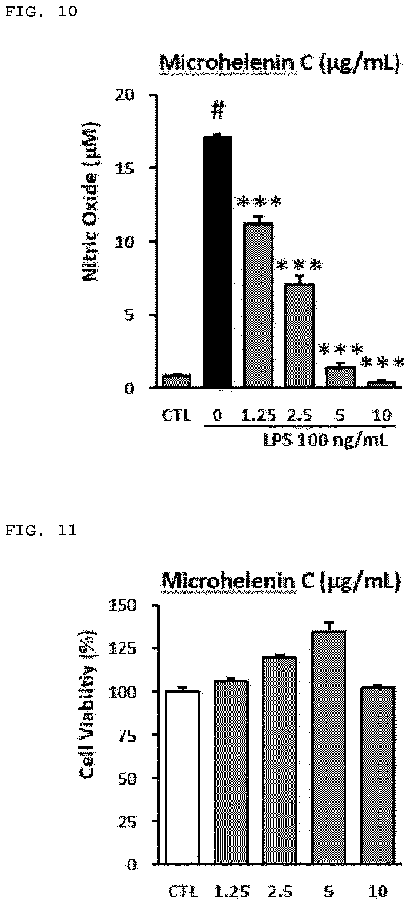

Referring to FIGS. 10 and 11, it could be seen that, as the concentration of Microhelenin C increased, the overall NO production decreased, but there was no significant change in cell viability, suggesting that Microhelenin C did not cause cytotoxicity. Specifically, it could be confirmed that, when the concentration of Microhelenin C was 10 pg/mL, the production of NO was the lowest and the cell viability was somewhat higher than that in the positive control group (CTL), indicating that Microhelenin C at this concentration was not cytotoxic.

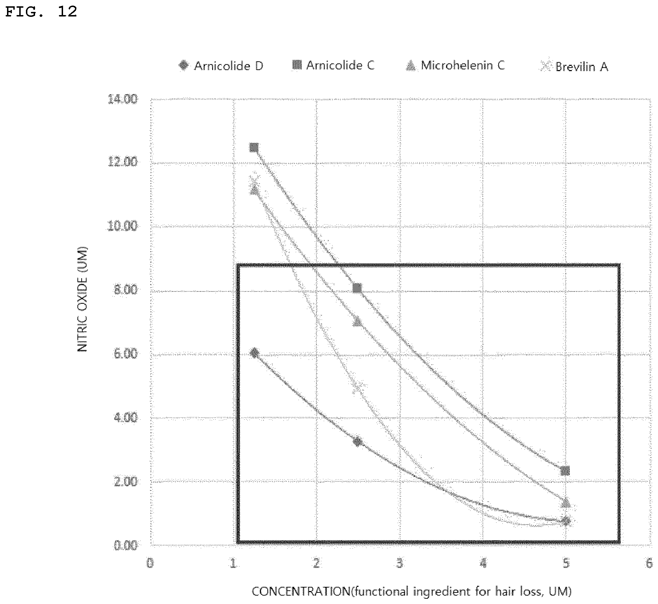

FIG. 12 is a graph showing, as potency values, the inflammation inhibition rates of Brevilin A, Arnicolide D, Arnicolide C, and Microhelenin C based on Experimental Example 2-1. Accordingly, the effects of Arnicolide D, Arnicolide C, and Microhelenin C relative to the same amount of Brevilin A are shown in Table 7 below.

**[Table 7]**

| Effect relative to the same amount | Arnicolide D | Arnicolide C | Microhelenin C | Brevilin A |
|---|---|---|---|---|
| Brevilin-A | 1.5 | 0.7 | 0.8 | 1.0 |

### 2-2. Anti-Inflammatory Effect and Cell Viability of Functional Ingredient for Hair Loss

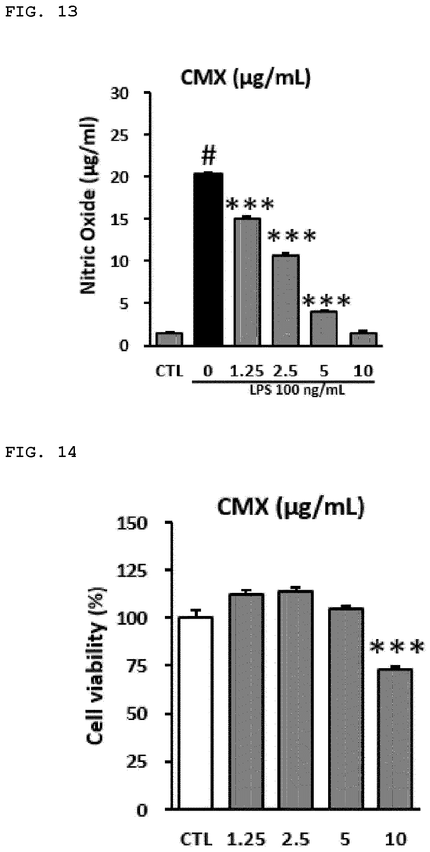

Referring to FIGS. 13 and 14, it could be seen that, as the concentration of CMX containing all of Brevilin A, Arnicolide D, Arnicolide C, and Microhelenin C increased, the overall NO production decreased, but there was no significant change in cell viability, indicating that CMX did not cause cytotoxicity. Specifically, it could be confirmed that, when the concentration of CMX was 10 pg/mL, the production of NO was the lowest and the difference in cell viability from that in the positive control group (CTL), suggesting that CMX at this concentration was not cytotoxic.

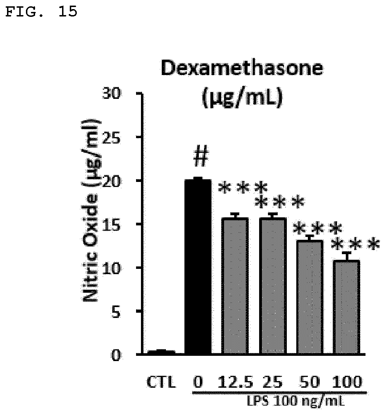

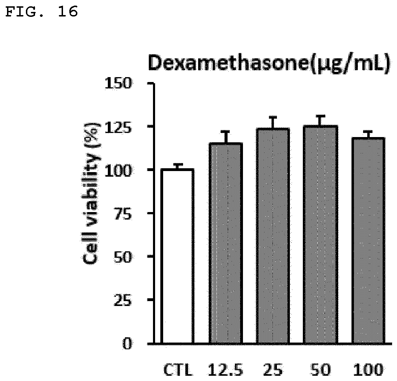

In addition, referring to FIGS. 15 and 16, it could be confirmed that, as the concentration of dexamethasone used for comparison increased, the overall NO production decreased, but there was no significant change in cell viability. Specifically, it could be confirmed that, compared to the case of CMX shown in FIG. 13, the reduction rate of NO production was somewhat low.

Therefore, it could be confirmed that, as the concentration of each of Brevilin A, Arnicolide D, Arnicolide C, and Microhelenin C increased, the overall NO production decreased, but no change in cell viability occurred, suggesting that each component did not cause cytotoxicity. Likewise, it could be confirmed that, as the concentration of CMX containing all of Brevilin A, Arnicolide D, Arnicolide C, and Microhelenin C increased, the overall NO production decreased and no change in cell viability occurred, suggesting that CMX did not cause cytotoxicity.

Accordingly, when the functional ingredient for hair loss (CMX) according to the present invention is contained, it is not cytotoxic and has a good anti-inflammatory effect. Specifically, when a cosmetic composition containing 5 to 50 wt% of CMX is prepared, it may be prepared as a stable formulation without causing layer separation, is not irritating to the skin, has high skin penetration rate, and is effective in alleviating hair loss.

## Claims

1. A functional composition for hair loss containing: a functional ingredient for hair loss; a diluent; a surfactant; a co-surfactant; a moisturizer; and an emulsifier.

2. The functional composition for hair loss according to claim 1, containing 5 to 50 wt% of the functional ingredient for hair loss, 10 to 30 wt% of the diluent, 20 to 50 wt% of the surfactant, 10 to 20 wt% of the co-surfactant, and 0.1 to 2 wt% of the emulsifier.

3. The functional composition for hair loss according to claim 1, wherein the functional ingredient for hair loss comprises, as an active ingredient, any one or more selected from the group consisting of Brevilin A, Arnicolide D, Arnicolide C, and Microhelenin C.

4. The functional composition for hair loss according to claim 1, wherein the functional ingredient for hair loss comprises, based on the total weight of the functional ingredient for hair loss, 10 to 90 wt% of Brevilin A, 0 to 35 wt% of Arnicolide D, 0 to 15 wt% of Arnicolide C, and 0 to 15 wt% of Microhelenin C.

5. The functional composition for hair loss according to claim 1, wherein the functional ingredient for hair loss comprises, based on the total weight of the functional ingredient for hair loss, 50 to 70 wt% of Brevilin A, 15 to 25 wt% of Arnicolide D, 0 to 15 wt% of Arnicolide C, and 0 to 15 wt% of Microhelenin C.

6. The functional composition for hair loss according to claim 1, wherein the functional ingredient for hair loss comprises, based on the total weight of the functional ingredient for hair loss, 50 to 70 wt% of Brevilin A, 15 to 25 wt% of Arnicolide D, 5 to 10 wt% of Arnicolide C, and 5 to 10 wt% of Microhelenin.

7. The functional composition for hair loss according to claim 1, wherein the diluent comprises any one or more selected from the group consisting of medium-chain triglyceride (MCT) oil, BRIJ 010-SS-(RB), Capryol 90, Capryol PGMC, Gantrez ES-435, Gantrez S-97 BF, Gelucire 43/01, Gelucire 44/14, Gelucire 50/13, Gelucire 48/16, Labrafac CC, Labrafac Lipophile WL1349, Labrafac PG, Labrafil M 1944 CS, Labrafil M 2125 CS, Labrafil M 2130 CS, Lauroglycol 90, Lauroglycol FCC, Monosteol, Peceol, Pharmasolve, Plurol Oleique CC497, Span 20-LQ-(SG), Span 60-PA-(SG), upper Refined Oleic Acid-LQ-(JP), Super Refined Tween 80A-LQ-(MH), Surfadone LP-300, TWEEN 60-SS-(SG), TWEEN 80 HP-LQ-(MH), TWEEN 80-LQ-(SG), and Vitamin E TPGS.

8. The functional composition for hair loss according to claim 1, wherein the surfactant comprises any one or more selected from the group consisting of Labrasol, Crodex A-PA-(RB), Geleol, Gelot 64, Suppocire AS2X, Suppocire BML, Synperonic PE/F 127, Synperonic PE/L 44, and Tefose 63.

9. The functional composition for hair loss according to claim 1, wherein the co-surfactant comprises any one or more selected from the group consisting of Transcutol, and Cremophor ELP.

10. The functional composition for hair loss according to claim 1, wherein the moisturizer comprises a polyol comprising any one or more selected from the group consisting of D-panthenol, L-panthenol, panthenoic acid, glycerin, butylene glycol, propylene glycol, polyethylene glycol, and sorbitol.

11. The functional composition for hair loss according to claim 1, wherein the emulsifier comprises a soybean-derived natural phospholipid comprising any one or more selected from the group consisting of lecithin, hydrogenated lecithin, unsaturated lecithin, and lysolecithin.

12. A cosmetic composition for preventing hair loss and promoting hair growth comprising the functional composition for hair loss according to any one of claims 1 to 9.

13. An agent for preventing and treating hair loss comprising the functional composition for hair loss according to any one of claims 1 to 9.
